# EUROPEAN PATENT SPECIFICATION

(11) **EP 1 565 445 B1**
(45) Date of publication and mention of the grant of the patent: **27.09.2006**
(21) Application number: 03811396.5
(22) Date of filing: 17.11.2003
(51) Int. Cl.: C07D 233/60, C07D 403/06, C07D 471/06, A61K 31/4174, A61K 31/4178, A61P 25/18

(54) **PROCESS FOR THE PREPARATION OF IMIDAZOLYL COMPOUNDS**
VERFAHREN ZUR HERSTELLUNG VON IMIDAZOLVERBINDUNGEN
PROCEDE DE PREPARATION DE COMPOSES IMIDAZOLYLE

(30) Priority: 18.11.2002 EP 02079838
(43) Date of publication of application: 24.08.2005
(73) Proprietor: Solvay Pharmaceuticals B.V., 1381 CP Weesp (NL)
(72) Inventor: VERBEEK, Jan-Maarten, NL-1381 CP Weesp (NL); VAN DER MEIJ, Paulus, F., C., NL-1381 CP Weesp (NL)
(74) Representative: Verhage, Marinus
(86) International application number: PCT/EP2003/050841
(87) International publication number: WO 2004/046116

(56) References cited:
- EP-B- 0 297 651
- DATABASE CAPLUS [Online] CHEMICAL ABSTRACTS SERVICE, COLUMBUS, OHIO, US; Database accession no. 1967:490636 XP002239462 & H. ANTAKI: "Synthetic routes to benz- and naphthindenoquinolines" JOURNAL OF THE CHEMICAL SOCIETY, SECTION C: ORGANIC CHEMISTRY., no. 17, 1967, pages 1581-1582, CHEMICAL SOCIETY. LETCHWORTH., GB ISSN: 0022-4952
- DATABASE CAPLUS [Online] CHEMICAL ABSTRACTS SERVICE, COLUMBUS, OHIO, US; Database accession no. 2001:557548 XP002239463 & A. G. KOREPIN ET AL.: "N-Substituted tetrahydro-1,3-oxazines and oxazolidines 1. A new version of the Mannich-reaction involving amino alcohols" RUSSIAN CHEMICAL BULLETIN., vol. 50, no. 1, 2001, pages 104-109, PLENUM PUBLISHING CO, NEW YORK, NY., US ISSN: 1066-5285

## Description

The present invention relates to a process for the preparation of imidazolyl compounds.

1,2,3,9-Tetrahydro-9-methyl-3-[2-methyl-1*H*-imidazol-1-yl)methyl]-4*H*-carbazol-4-one (ondansetron) is known from EP191562 and US4,695,578. In these patent publications a general dass of compounds including ondansetron and homologous compounds, their preparation and their use as potent selective antagonists at "neuronal" 5-hydroxytryptamine receptors and their use in the treatment of migraine and psychotic disorders is described.

(10R)-5,6,9,10-Tetrahydro-10-[(2-methyl-1*H*-imidazol-1-yl)methyl]-4*H*-pyrido[3,2,1-jk]-carbazol-11(8*H*)-one (cilansetron) (also known as (R)-(-)-4,5,6,8,9,10-hexahydro-10-[(2-methyl-1H-imidazol-1-yl)methyl]-11H-pyrido-[3,2,1-jk]-carbazol-11-one) is known from EP-B-0297651, from EP-B-0601345 and from EP-B-768309. In the first patent publication a general class of compounds, including cilansetron and homologous compounds, their preparation and their use as 5-HT antagonists is described. The second patent publication describes the use of a selection of these type of compounds for the treatment of certain diseases and the third the preparation of enantiomerically pure compounds and their hydrochloride monohydrate.

It is a common feature of the above compounds that they contain a substituted imidazolyl group attached to the α-place with regard to the keto group of the carbazole system with a methylene bridge. Several possibilities for the synthesis of these compounds are described in the mentioned patent publications. It is a common feature in these syntheses that the substituted imidazolyl group is introduced by means of a Mannich reaction, followed by a deamination to yield an intermediate exomethylene compound which is reacted with the substituted imidazolyl group (see scheme 1 for an example).

A drawback of this route is that the yield in this sequence of reaction steps is rather low. In US 4,695,578 the first step which is normally giving the lowest yield is not described and the second step (Example 7 of US 4,695,578) gives a yield of 68%. In EP-B-0297651 the first step (Example 1c of EP-B-0297651) has a yield of 53% and the second step (Example 1d of EP-B-0297651) a yield of 87%. During scale-up it appeared that this route gives rise to the formation of a considerable amount of tar-like side-products.

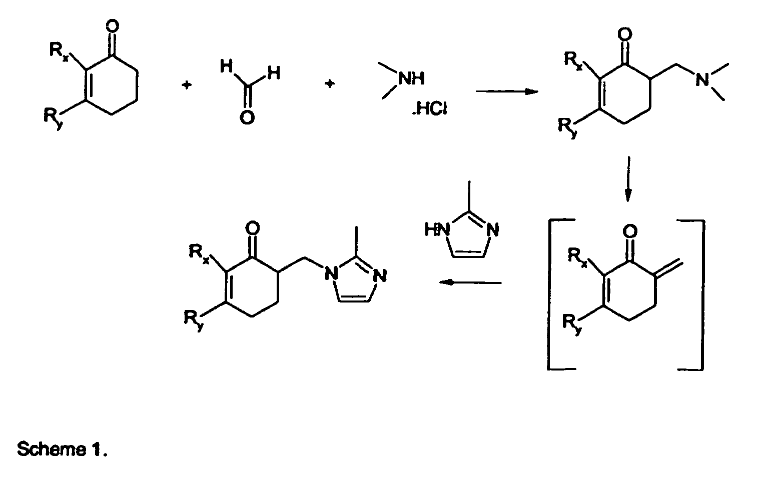

It is the objective of the present invention to provide an alternative process to prepare imidazolyl-compounds which process should be economically operative and meet one or more of the following requirements: a) a relatively high yield, b) short reaction times compared with prior art processes, c) less side reactions, d) higher quality of the final product and e) using non-diluted reaction conditions and an environmentally acceptable solvent

It has surprisingly been found that these type of imidazolyl compounds can easily be prepared using a substituted oxazolidine compound for the introduction of the methylene bridge.

Therefore the present invention relates to a method for the preparation of an imidazolyl compound of the general formula 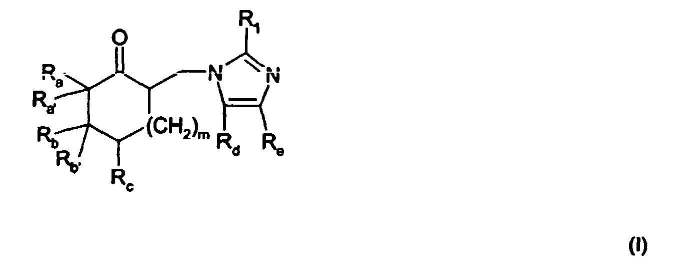 wherein:
Rₐ and R_{b} each separately are (C₁-C₆)alkyl, (C₁-C₆) alkoxyalkyl, optionally substituted aryl or heteroaryl;
   or wherein Rₐ and R_{b} together form a further homocyclic or heterocyclic system comprising one or more rings;
R_{a'} and R_{b'} each are hydrogen or together form a carbon-carbon double bond, said carbon-carbon double bond optionally being part of an aromatic system;
R_{c} is hydrogen, (C₁-C₆)alkyl, (C₁-C₆)alkoxy, (C₁-C₆)alkoxyalkyl or halogen;
R_{d} is hydrogen or (C₁-C₄)alkyl;
Rₑ is hydrogen or (C₁-C₄)alkyl;
m is 1 or 2; and
R₁ is hydrogen or (C₁-C₄)alkyl;
as well as its acid addition salt
characterized in that a compound of the general formula 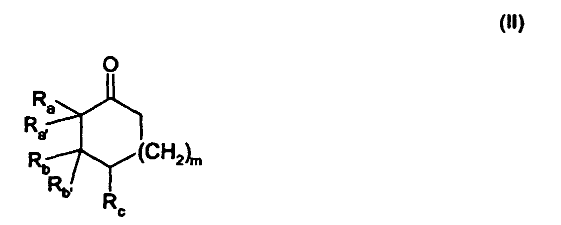 is reacted with a compound of the formula 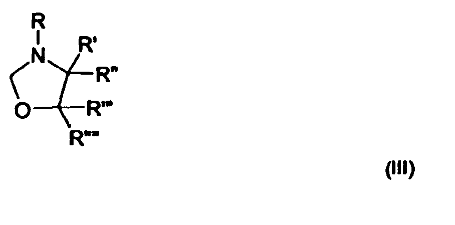 wherein:
R is a hydrogen, a (C₁-C₄)alkyl group optionally substituted with a hydroxygroup or an optionally substituted aryl group,
R', R", R‴ and R"" each individually are a hydrogen or a (C₁-C₄)alkyl group; followed by a reaction with a compound of the formula 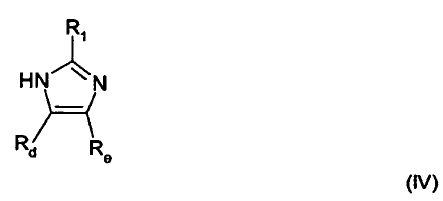
wherein R₁, R_{d} and Rₑ have the meanings defined above;
and optionally followed by a reaction with a suitable acid.

Alkyl groups of the present invention include straight-chained, branched and cydic alkyl radicals containing up to 6 carbon atoms. Suitable alkyl groups may be saturated or unsaturated. Further, an alkyl group may also be substituted one or more times with substituents selected from the group consisting of aryl, halo, hydroxy, cyano or one- or di-alkyl substituted amino.
Aryl groups of the present invention include aryl radicals which may contain up to 6 hetero atoms. An aryl group may also be optionally substituted one or more times with an substituent selected from the group consisting of aryl, (C₁-C₆) alkyl, halo, hydroxy, cyano or one- or di-alkyl substituted amino, and it may be also fused with an aryl group or cycloalkyl rings. Suitable aryl groups include, e.g. phenyl, naphtyl, tolyl, imidazolyl, pyridyl, pyrroyl, thienyl, pyrimidyl, thiazolyl and furyl groups.
With a homocyclic system is meant a system containing at least one saturated or unsaturated cyclic group containing only carbon atoms and hydrogen atoms.
With a heterocyclic system is meant a system containing at least one saturated or unsaturated cydic group containing also one or more heteroatoms such as N, O or S.
Both the homocyclic and heterocyclic system may optionally be substituted with a substituent selected from the group consisting of alkyl, aryl , cyano, halogen, hydroxy or one- or di-alkyl substituted amino.

In a preferred embodiment of the invention Rₑ is hydrogen or (C₁-C₆)alkyl, R_{d} is hydrogen or (C₁-C₄)alkyl; Rₑ is hydrogen or (C₁-C₄)alkyl; and R₁ is hydrogen, methyl or ethyl.

The reaction according to the invention is especially useful for the preparation of compounds of the general formula 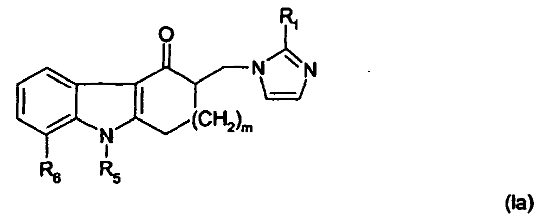 wherein:
m is 1 or 2;
R₁ is hydrogen, methyl or ethyl; and
R₅ is a (C₁-C₄) alkyl;
R₆ is a hydrogen or a (C₁-C₄)alky), or
R₅ and R₆ together with the intermediate atoms form a 5, 6, or 7 membered ring, optionally substituted with one or two substituents selected from the group consisting of halogen, hydroxy, (C₁-C₄)alkyl, (C₁-C₄)alkoxyalkyl and (C₁₋C₄)alkoxy.

In this case the starting compound is a compound of the general formula

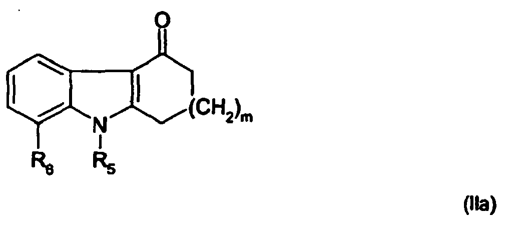

This compound is further referred to as a carbazolone compound.

Preferred compounds of the general formula la are the compound wherein m=1 and R₅ and R₆ together with the intermediate atoms form a 6-membered ring and the compound wherein m=1, R₅ is methyl and R₆ is hydrogen. For the first compound the yield for the process starting with 5,6.9,10-tetrahydro-4*H*-pyrido[3,2,1-jk]carbazol-11(8*H*)-one and 3-oxazolidineethanol is 77% (see Example 2) compared with the overall yield of 46% in the process according to EP0297651 (Examples lc and ld). Higher yields may be obtained at a production scale.

In the substituted oxazolidine preferably one of the R' and R" and one of the R" and R"" is hydrogen, as an oxazolidine disubstituted on the same carbon atom, such as 4,4-dimethyloxazolidine gives a lower yield in the reaction. Preferred oxazolidines are 3-oxazolidineethanol and 3-ethyl-oxazolidine. The most preferred oxazolidine is 3-oxazolidineethanol.

The reaction is carried out in acidic medium, and the grade of acidity depends on the activation of the system that has to react. In the case of carbazolone systems the medium should be highly acidic. Examples of suitable acids in the last case are methanesulfonic acid, trifluoromethanesulfonic acid, p-toluenesulfonic acid and HCl gas in alcoholic medium.

In order to get a high yield, the reaction solution should contain only a low amount of water. The amount of water should preferably be below 0.6% (V/V), more preferably below 0.3 % V/V and most preferably below 0.1% V/V.

The optimal reaction temperature is dependent on the starting material and the solvent and differs for the two reaction steps. The first step of the reaction can be performed between 40°C and 110°C. For the carbazolone systems the preferred reaction temperature in the first step is between 50°C and 90°C and the most preferred temperature is approximately 70°C. The second step can generally be performed between 100°C and 140°C. For the carbazolone systems the preferred reaction temperature In the second step is between 110°C and 130°C and the most preferred temperature is approximately 120°C.

The reaction can be performed in different solvents such as dipolar aprotic solvents like DMF or in alcohols. Preferred solvents are C₄-C₇, alcohols and the choice may depend on the desired reaction temperature. Examples of suitable alcohols are 1-butanol, 1-hexanol and isoamyl alcohol. A preferred alcohol is 1-butanol. Also suitable are mixtures of aromatic hydrocarbons and alcohols, such as mixtures of toluene and an alcohol and monochlorobenzene and an alcohol. A preferred mixture is a mixture of monochlorobenzene and methanol. When solvent mixtures are used the lower boiling solvent can be distilled off before the second step in order to reach higher reflux temperatures of the solvent system in the second step.

The ratio of the solvent volume to the amount of reactants in the mixture can be varied over a relatively broad range and depends on the solubility of the reactants. In general the ratio of the amount of solvent to the amount of reactants can typically be about 1 : 1 to 15 :1, where the ratio is expressed as the volume of solvent relative to the weight of the reactants in the solvent (in ml/g). Preferably the ratio is about 1:1 to about 10 :1. in the case of the carbazolone systems the preferred ratio of the volume of solvent to the weight of reactants is about 4:1.

The products obtained can be crystallized from different solvents. Examples of solvents for the cyrstallisation of free bases are aromatic hydrocarbons such as toluene. The hydrochloric acid salts can e.g. be crystallized from alcoholic solvents, such as isopropanol or 1-butanol.

The following examples are only intended to further illustrate the invention, in more detail, and therefore these examples are not deemed to restrict the scope of the invention in any way.

### Example 1: Materials and Methods

5,6,9,10-Tetrahydro-4*H*-pyrido[3,2,1-jk]carbazol-11(8*H*)-one was made according to EP0375045. 3,4 -Dihydro-1(2*H*)-naphthalenone was obtained from a commercial source. 1,2,3,9-Tetrahydro-9-methyl-4H-carbazol-4-one was made according to US 3,892,766 from Wamer-Lambert Company and Elz, S. and Heil, W., Bioorganic & Medicinal Chemistry Letters 1995, 5, 667-672. Methanesulfonic acid was obtained from a commercial source.
NMR spectra were measured on a Varian VXR 200 and MS spectra on a Finnigan TSQ 7000. HPLC analyses were performed on a HP1050 system with a Separations 757 detector (250 nm) and a Separations Marathon XT column oven at 35 °C. The column used was a Zorbax XDB C8 colomn 15x0.3 cm. The eluens was prepared as follows: mix 2 l water, 2 ml triethylemine and 5 ml 25% ammonia, buffer it at pH=4 with formic acid and add 0.5 l acetonitril. The flow was 1 ml/min.

### Example 1a: Preparation of oxazolidines.

### 3-Oxazolidineethanol was made as follows:

Equimolair amounts of diethanolamine and paraformaldehyde in 1-butanol were heated to 70°C. After 1 hour reaction time the water formed was removed by azeotropic distillation with 1-butanol.

3-Ethyl-oxazolidine was made according to Heany, H. et al., Tetrahedron 1997, 53, 14381-96.

4,4-Dimethyl-oxazolidine is commercially available and was purchased as a 75% w/w solution in water. The 4,4-dimethyl-oxazolidine was extracted from the water layer by washing with dichloromethane/ saturated NaCl-solution. The dichloromethane layer was dried on anhydrous sodium sulfate and subsequently evaporated.

### Example 2. Reaction of 5,6,9,10-tetrahydro-4H-pyrido[3,2,1-jk]carbazol-11(8H)-one with 3-oxazolidineethanol.

5,6,9,10-tetrahydro-4*H*-pyrido[3,2,1-jk]carbazol-11(8*H*)-one (25.00 g ≡ 111.0 mmole) and methanesulfonic acid (17.06 g ≡ 177.5 mmole) in 1-butanol (100 ml) were heated to 70°C. In 3 minutes a solution was added of 3-oxazolidineethanol (19.49 g ≡ 166.4 mmole) in 1-butanol (39 ml).
After 50 minutes at 80°C 2-methylimidazole (45.55 g ≡ 554.8 mmole) and 1-butanol (10 ml) were added. After 1.5 hours at 120°C the reaction mixture was partly evaporated till 30 ml of 1-butanol was left over.
At 70°C, 75 ml of toluene and 50 ml of water were added to the residue. The layers were separated. The water layer was extracted with 75 ml of toluene. The combined toluene layers were washed three times with 100 ml of water.
The organic layer was evaporated to dryness and subsequently 125 ml of 1-butanol was added. To the resulting solution 12.5 ml of 36% m/m hydrochloric acid was added. After stirring for 2 hours at room temperature the formed sol id was filtered off and washed with 1-butanol and MTBE. Yield after drying: 30.40 g (77.0%) 5,6,9,10-tetrahydro-10-[(2-methyl-1*H*-imidazol-1-yl)methyl]-4H-pyrido[3,2,1-jk]carbazol-11(8*H*)-one hydrochloride (77.0%). HPLC: ≥ 95%. ¹H NMR [200 MHz, DMSO-d⁸:CDCl₃4:1] δ 1.97(1H,m), 2.18 (3H,m), 2.68(3H,s), 2.95(2H,t), 3.00(1H,dd), 3.12(2H,m), 4.13(2H,m), 4.29(1H,dd), 4.66(1H,dd), 6.97(1H,d), 7.09(1H,t), 7.55(1H,d), 7.68(1H,d) and 7.71(1H,d), MS [ESI] MH⁺ = 320.

### Example 3. Reaction of 5,6,9,10-tetrahydro-4H-pyrido[3,2,1-jk]carbazol-11(8H)-one with 4,4-dimethyl-oxazolidine.

5,6,9,10-tetrahydro-4*H*-pyrido[3,2,1-jk]carbazol-11(8*H*)-one (20.00 g ≡ 88.8 mmole) and methanesulfonic acid (13.65 g ≡ 142.0 mmole) in 1-butanol (60 ml) were heated to 70°C. In 2 minutes 4,4-dimethyl-oxazolidine (13.47 g ≡ 133.2 mmole) in 1-butanol (10 ml) was added.
After 50 minutes at 80°C 2-methylimidazole (36.45 g ≡ 444.0 mmole) and butanol (10 ml) were added. After 2 hours at 120°C the reaction mixture was partly evaporated till 20 ml of 1-butanol was left over.
At 70°C, 60 ml of toluene and 40 ml of water were added to the residue. The layers were separated. The water layer was extracted with 60 ml of toluene. The combined toluene layers were washed three times with 80 ml of water.
The organic layer was evaporated to dryness and subsequently 100 ml of 1-butanol was added. To the resulting solution 10.0 ml of 36% m/m hydrochloric acid was added. After stirring for 2 hours at room temperature the formed so lid was filtered off and washed with 1-butanol and MTBE. Yield after drying: 12.38 g 5,6,9,10-tetrahydro-10-[(2-methyl-1*H*-imidazol-1-yl)-methyl]-4*H*-pyrido[3,2,1-jk]carbazol-11(8H)-one hydrochloride (39.2%). HPLC: ≥ 95%. ¹H NMR and MS: see Example 2. The mother liquor contained 3.45 g (10.9%) of product.

### Example 4. Reaction of 5,6,9,10-tetrahydro-4H-pyrido[3,2,1-jk]carbazol-11(8H)-one with 3-ethyl-oxazolidine.

5,6,9,10-tetrahydro-4H-pyrido[3,2,1-jk]carbazol-11(8H)-one (20.00 g ≡ 88.8 mmole) and methanesulfonic acid (13.65 g≡ 142.0 mmole) in 1-butanol (60 ml) were heated to 70°C. In 2 minutes 3-ethyl-oxazolidine (13.46 g ≡ 133.2 mmole) in 1-butanol (10 ml) was added.
After 50 minutes at 80°C 2-methylimidazole (36.45 g ≡ 444.0 mmole) and 1-butanol (10 ml) were added. After 2 hours at 120°C the reaction mixture was partly evaporated till 20 ml of 1-butanol was left over.
At 70°C, 60 ml of toluene and 40 ml of water were added to the residue. The layers were separated. The water layer was extracted with 60 ml of toluene. The combined toluene layers were washed three times with 80 ml of water.

The organic layer was evaporated to dryness and subsequently 100 ml of 1 -butanol was added. To the resulting solution 10.0 ml of 36% m/m hydrochloric acid was added. After stirring for 2 hours at room temperature the formed solid was filtered off and washed with 1-butanol and MTBE. Yield after drying: 22.10 g (70.0 %) 5,6,9,10-tetrahydro-10-[(2-methyl-1*H*-imidazol-1-yl)methyl]-4H-pyrido[3,2,1-jk]carbazol-11(8*H*)-one hydrochloride (70.0%). HPLC: ≥ 95%. ¹H NMR and MS: see Example 2.

### Example 5. Reaction of 3,4-dihydro-1(2H)-naphthalenone with 3-oxazolidineethanol.

3,4-dihydro-1(2*H*)-naphthalenone (12.98 g ≡ 88.8 mmole) and methanesulfonic acid (13.65 g ≡ 142.0 mmole) in 1-butanol (60 ml) were heated to 50°C. In 2 minutes a solution was added of 3-oxazolidineethanol (15.59 g ≡ 133.1 mmole) in 1-butanol (14 ml).

After 50 minutes at 80°C 2-methylimidazole (36.45 g ≡ 444.0 mmole) and 1-butanol (10 ml) were added. After 2 hours at 120°C the reaction mixture was partly evaporated till 20 ml of 1-butanol was left over.
At 70°C, 60 ml of toluene and 40 ml of water were added to the residue. The layers were separated. The water layer was extracted with 60 ml of toluene. The combined toluene layers were washed three times with 80 ml of water.
The organic layer was evaporated to dryness and subsequently 100 ml of 1-butanol was added. To the resulting solution 10.0 ml of 36% m/m hydrochloric acid was added. The resulting solution was evaporated till an end volume of 60 ml. After stirring for 2 hours at room temperature the formed solid was filtered off and washed with 1-butanol and MTBE. Yield after drying: 15.28 g (62.2%) 3,4-dihydro-2-[(2-methyl-1H-imidazol-1-yl)methyl]-1(2H)-naphthalenone hydrochloride. HPLC: ≥ 95% . ¹H NMR[200 MHz, DMSO-d⁶:CDCl₃4:1] δ 2.00 (2H,m), 2.73 (3H,s), 3.20 (3H,m), 4.27 (1H,dd), 4.68 (1H,dd), 7.35 (2H,t), 7.55 (2H,m), 7.70 (1H,d) and 7.90 (1H,d). MS [ESI] MH⁺ = 241. The mother liquor contained 3.28 g (13.3%) of product.

### Example 6. Reaction of 3,4-dihydro-(2H)-naphthalenone with 4,4-dimethyl-oxazolidine.

3,4-dihydro-1(2*H*)-naphthalenone (12.98 g ≡ 88.8 mmole) and methanesulfonic acid (13.65 g ≡ 142.0 mmole) in 1-butanol (60 ml) were heated to 70°C. In 2 minutes 4,4-dimethyl-oxazolidine (13.46 g ≡ 133.1 mmole) in 1-butanol (10 ml) was added. After 50 minutes at 80°C 2-methylimidazole (36.45 g ≡ 444.0 mmole) and 1-butanol (10 ml) were added. After 2 hours at 120°C the reaction mixture was partly evaporated till 20 ml of 1-butanol was left over.
At 70°C, 60 ml of toluene and 40 ml of water were added to the residue. The layers were separated. The water layer was extracted with 60 ml of toluene. The combined toluene layers were washed three times with 80 ml of water.
The organic layer was evaporated to dryness and subsequently 100 ml of 1-butanol was added. To the resulting solution 10.0 ml of 36% m/m hydrochloric acid was added. The resulting solution was evaporated till an end volume of 50 ml. After stirring for 2 hours at 0°C the formed solid was filtered off and washed with 1-butanol and MTBE Yield after drying: 14.13 g (57.5%) 3,4-dihydro-2-[(2-methyl-1H-imidazol-1-yl)methyl]-1(2*H*)-naphthalenone hydrochloride. HPLC: ≥ 95%. ¹H NMR and MS: see Example 5. The mother liquor contained 2.33 g (9.5%) of product.

### Example 7. Reaction of 3,4-dihydro-1(2H)-naphthalenone with 3-ethyl-oxazolidine.

3,4-dihydro-1(2*H*)-naphthalenone (12.98 g ≡ 88.8 mmole) and methanesulfonic add (13.65 g ≡ 142.0 mmole) in 1-butanol (60 ml) were heated to 50°C. In 2 minutes 3-ethyl-oxazolidine (13.46 g ≡ 133.1 mmole) in 1-butanol (10 ml) was added. After 50 minutes at 80°C 2-methylimidazole (36.45 g ≡ 444.0 mmole) and 1-butanol (10 ml) were added. After 2 hours at 120°C the reaction mixture was partly evaporated till 20 ml of 1-butanol was left over.
At 70°C, 60 ml of toluene and 40 ml of water were added to the residue. The layers were separated. The water layer was extracted with 60 ml of toluene. The combined toluene layers were washed three times with 80 ml of water.
The organic layer was evaporated to dryness and subsequently 100 ml of 1-butanol was added. To the resulting solution 10.0 ml of 36% m/m hydrochloric acid was added. The resulting solution was evaporated till an end volume of 50 ml. After stirring for 2 hours at 0°C the formed solid was filtered off and washed with 1-butanol and MTBE. Yield after drying: 17.30 g 3,4-dihydro-2-[(2-methyl-1*H*-imidazol-1-yl)methyl]-1(2*H*)-naphthalenone hydrochloride (70.4%). HPLC: ≥ 95%. ¹H NMR and MS: see Example 5.

### Example 8. Reaction of 1,2,3,9-tetrahydro-9-methyt-4H-carbazol-4-one with 3-oxazolidineethanol.

1,2,3,9-tetrahydro-9-methyl-4H-carbazol-4-one (13.26 g ≡ 66.5 mmole) and methanesulfonic acid (10.23 g ≡ 106.4 mmole) in 1-butanol (45 ml) were heated to 90°C. In 2 minutes 11.68 g (99.8 mmole) of 3-oxazolidineethanol in 1-butanol (11 ml) was added.
After 50 minutes at 80°C 2-methylimidazole (27.32 g ≡ 332.5 mmole) and 1-butanol (8 ml) were added. After 2 hours at 120°C 180 ml of toluene and 120 ml of water were added at 80°C. The layers were separated. The water layer was extracted with 180 ml of toluene and 60 ml of 1-butanol. The combined organic layers were washed twice with 240 ml of water. The organic layer was evaporated to dryness. 150 ml of 1-butanol and 10 ml of 36% m/m hydrochloric acid were added to the residue. At 0 °C crystallization soon occurred. After 1 hour at 0°C the formed crystals were filtered off, washed with 1-butanol and MTBE and subsequently dried: 15.39 g (70.1%) of 1,2,3,9-tetrahydro-9-methyl-3-[(2-methyl-1*H*-imidazol-1-yl)methyl]-4*H*-carbazol-4-one hydrochloride was isolated. HPLC: ≥ 95%. ¹H NMR [200 MHz, DMSO-d⁶:CDCl₃ 4:1] δ 2.00 (1H,m), 2.20 (1H,m), 3.69 (3H,s), 3.09 (3H,m), 3.75 (3H,s), 4.30 (1H,dd), 4.67 (1H,dd), 7.23 (2H,m), 7.53 (2H,m), 7.69 (1H,d), 8.01 (1H, d). MS [ESI] MH⁺ = 294. The mother liquor contained 3.19 g (14.5%) of product

### Example 9. Reaction of 1,2,3,9-tetrahydro-9-methyl-4H-carbazol-4-one with 4,4-dimethyl-oxazolidine.

1,2,3,9-tetrahydro-9-methyl-4H-carbazol-4-one (13.26 g = 66.5 mmole) and methanesulfonic acid (10.23 g ≡ 106.4 mmole) in 1-butanol (45 ml) were heated to 90°C. In 2 minutes 4,4-dimethyl-oxazoildine (10.09 g ≡ 99.9 mmole) in 1-butanol (8 ml) was added.
After 50 minutes at 80°C 2-methylimidazole (27.32 g ≡ 332.5 mmole) and 1-butanol (8 ml) were added. After 2 hours at 120°C 180 ml of toluene and 120 ml of water were added at 80°C. The layers were separated. The water layer was extracted with 180 ml of toluene and 60 ml of 1-butanol. The combined organic layers were washed twice with 240 ml of water. The organic layer was evaporated to dryness. 150 ml of 1-butanol and 10 ml of 36% m/m hydrochloric add were added to the residue. At 0°C crystallization soon occurred. After 1 hour at 0°C the formed crystals were filtered off, washed with 1-butanol and MTBE and subsequently dried: 10.02 g (45.7%) of 1,2,3,9-tetrahydro-9-methyl-3-[(2-methyl-1*H-*imidazol-1-yl)methyl]-4*H*-carbazol-4-one hydrochloride. The mother liquor contained 2.70 g (12.3%) of product.
HPLC: ≥ 95%. NMR and MS: see Example 8.

### Example 10. Reaction of 1,2,3,9-tetrahydro-9-methyl-4H-carbazol-4-one with 3-ethyl-oxazolidine.

1,2,3,9-tetrahydro-9-methyl-4*H*-carbazol-4-one (13.26 g ≡ 66.5 mmole) and methanesulfonic acid (10.23 g ≡ 106.4 mmole) in 1-butanol (45 ml) were heated to 90°C. In 2 minutes 3-ethyl-oxazolidine (10.09 g ≡ 99.9 mmole) in 1-butanol (8 ml) was added. After 50 minutes at 80°C 2-methylimidazole (27.32 g ≡ 332.5 mmole) and 1-butanol (8 ml) were added. After 2 hours at 120°C 180 ml of toluene and 120 ml of water were added at 80°C. The layers were separated. The water layer was extracted with 180 ml of toluene and 60 ml of 1-butanol. The combined organic layers were washed twice with 240 ml of water. The organic layer was evaporated to dryness. 150 ml of 1-butanol and 10 ml of 36% m/m hydrochloric acid were added to the residue. At 0 °C crystallization soon occurred. After 1 hour at 0°C the formed crystals were filtered off, washed with 1-butanol and MTBE and subsequently dried: 15.67 g (71. 4%) of 1,2,3,9-tetrahydro-9-methyl-3-[(2-methyl-1*H*-imidazol-1-yl)methyl]-4*H*-carbazol-4-one hydrochloride was isolated. HPLC: ≥95%. NMR and MS: see Example 8. The mother liquor contained 2.06 g (9.4%) of product.

## Claims

1. Method for the preparation of an imidazolyl compound of the general formula 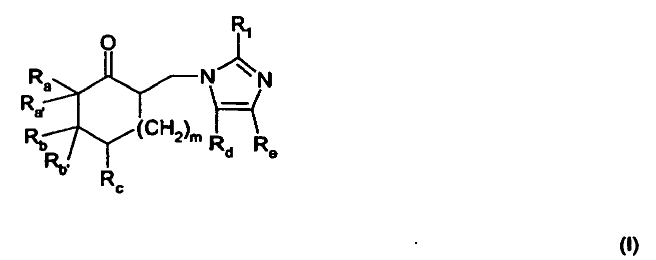 wherein:
Rₐ and R_{b} each separately are (C₁-C₆)alkyl, (C₁-C₆)alkoxyalkyl, optionally substituted aryl or heteroaryl;
or wherein Rₐ and R_{b} together form a further homocyclic or heterocyclic system comprising one or more rings;
R_{a'} and R_{b'} each are hydrogen or together form a carbon-carbon double bond, said carbon-carbon double bond optionally being part of an aromatic system;
R_{c} is hydrogen, (C₁-C₆)alky, (C₁-C₆)alkoxy, (C₁-C₆)alkoxyalkyl or halogen;
R_{d} is hydrogen or (C₁-C₄)alkyl;
Rₑ is hydrogen or (C₁-C₄)alkyl;
m is 1 or 2; and
R₁ is hydrogen or (C₁-C₄)alkyl;
as well as its acid addition salt;
**characterized in that** a compound of the general formula 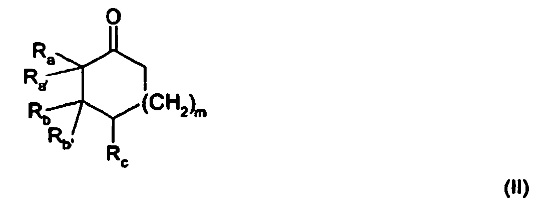 wherein Rₐ, R_{a'} , R_{b} and R_{b'} have the meanings defined above;
is reacted with a compound of the formula 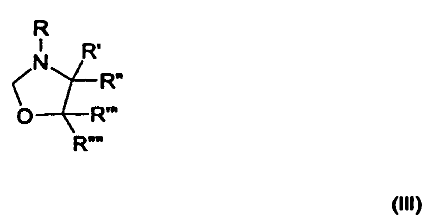 wherein:
R is a hydrogen, a (C₁-C₄)alkyl group optionally substituted with a hydroxygroup or an optionally substituted aryl group,
R', R", R‴ and R"" each individually are a hydrogen or a (C₁-C₄)alkyl group; followed by a reaction with a compound of the formula 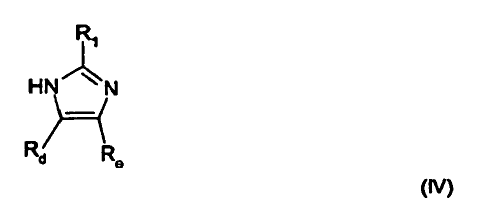
wherein R₁, R_{d} and Rₑ have the meanings defined above;
and optionally followed by a reaction with a suitable add

2. Method according to claim 1, wherein
Rₐ, R_{b}, R_{a'}, R_{b'}, R, R', R", R‴ and R"" have the same meanings as in claim 1;
R_{c} is hydrogen or (C₁-C₆)alkyl;
R_{d} is hydrogen or (C₁-C₄)alkyl;
Rₑ is hydrogen or (C₁-C₄)alkyl;
m is 1 or 2; and
R₁ is hydrogen, methyl of ethyl.

3. Method according to claim 1-2 for the preparation of an imidazolyl compound of the general formula 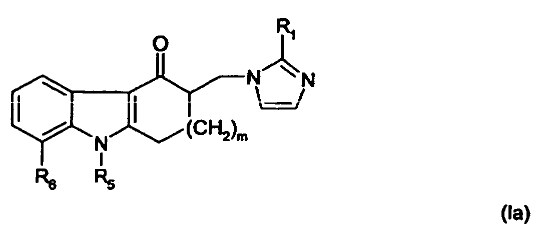 wherein:
m is 1 or 2;
R₁ is hydrogen, methyl or ethyl; and
R₅ is a (C₁-C₄)alkyl
R₆ is a hydrogen or a (C₁-C₄)alkyl, or
R₅ and R₆ together with the intermediate atoms form a 5, 6, or 7 membered ring, optionally substituted with one or two substituents selected from the group consisting of halogen, hydroxyl, (C₁-C₄)alkyl, (C₁-C₄)alkoxyalkyl and (C₁-C₄)alkoxy
as well as its pharmaceutically acceptable add addition salt;
**characterized in that** a compound of the general formula 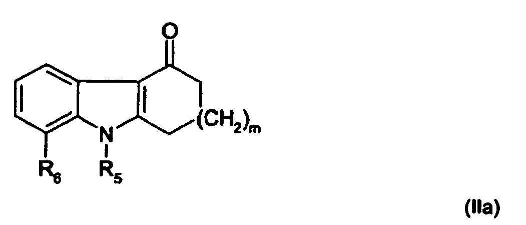 wherein R₅, R₆ and m have the meanings defined above;
is reacted with a compound of the formula 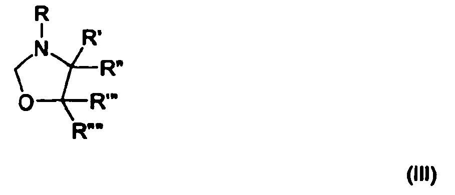 wherein R, R', R", R‴ and R"" have the same meanings as in claim 1;
followed by a reaction with a compound of the formula 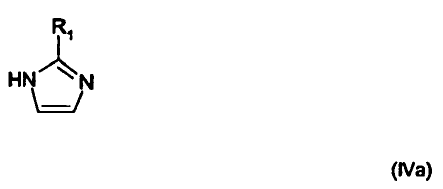 wherein R₁ has the same meaning as in claim 1.

4. Method according to claims 1-2, **characterized in that** R, R', R", R‴ and R'" in formula (III) are 2-hydroxyethyl, hydrogen, hydrogen, hydrogen and hydrogen respectively.

5. Method as claimed in all preceding claims, **characterized in that** m=1 and that R₅ and R₆ together with the intermediate atoms form a 6-membered ring.

6. Method as claimed in daims 1-4, **characterized in that** m=1, that R₅ is methyl and that R₆ is hydrogen.

7. Method as claimed in any of the preceding claims, **characterized in that** the reaction is performed in an alcoholic solvent

8. Method as claimed in claim 7, **characterized in that** the alcoholic solvent is 1-butanol.

9. Method as claimed in claims 1-6, **characterized in that** the reaction is performed in a mixture of an alcoholic solvent and an aromatic hydrocarbon

10. Method as claimed in claim 9, **characterized in that** said mixture is a mixture of methanol and chlorobenzene.

## Patentansprüche

1. Verfahren für die Herstellung einer Imidazolylverbindung der allgemeinen Formel 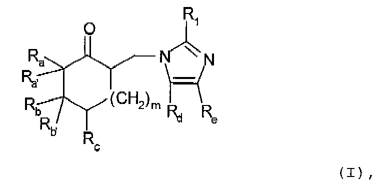 worin:
Rₐ und R_{b} jeweils getrennt für (C₁-C₆)Alkyl, (C₁-C₆)Alkoxyalkyl, gegebenenfalls substituiertes Aryl oder Heteroaryl stehen;
oder worin Rₐ und R_{b} zusammen ein weiteres homocyclisches oder heterocyclisches System bilden, welches einen oder mehrere Ringe umfasst;
Rₐ, und R_{b'} jeweils Wasserstoff darstellen oder zusammen eine Kohlenstoff-Kohlenstoff-Doppelbindung bilden, wobei besagte Kohlenstoff-Kohlenstoff-Doppelbindung gegebenenfalls Teil eines aromatischen Systems ist;
R_{c} Wasserstoff, (C₁-C₆)Alkyl, (C₁-C₆)Alkoxy, (C₁-C₆)Alkoxyalkyl oder Halogen darstellt;
R_{d} Wasserstoff oder (C₁-C₄)Alkyl darstellt;
Rₑ Wasserstoff oder (C₁-C₄)Alkyl darstellt;
m 1 oder 2 ist; und
R₁ Wasserstoff oder (C₁-C₄)Alkyl darstellt; als auch ihres Säureadditionssalzes;
**dadurch gekennzeichnet, dass** eine Verbindung der allgemeinen Formel 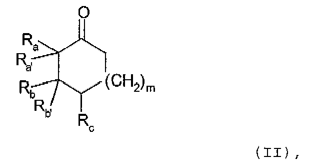 worin Rₐ, R_{a'}, R_{b} und R_{b'} die oben definierten Bedeutungen haben; umgesetzt wird mit einer Verbindung der Formel 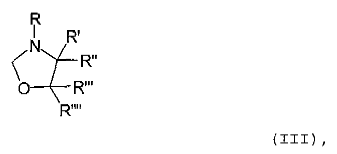 worin:
R ein Wasserstoff, eine (C₁-C₄)Alkylgruppe, gegebenenfalls substituiert mit einer Hydroxygruppe, oder eine gegebenenfalls substituierte Arylgruppe darstellt,
R', R", R‴ und R"" jeweils einzeln ein Wasserstoff oder eine (C₁-C₄)Alkylgruppe darstellen; gefolgt von einer Umsetzung mit einer Verbindung der Formel 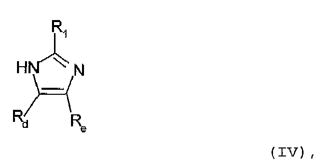
worin R₁, R_{d} und Rₑ die oben definierten Bedeutungen haben; und gegebenenfalls gefolgt von einer Umsetzung mit einer geeigneten Säure.

2. Verfahren gemäß Anspruch 1, worin
Rₐ, R_{b}, R_{a'} , R_{b'} , R, R', R", R‴ und R"" die gleichen Bedeutungen wie in Anspruch 1 haben;
R_{c} Wasserstoff oder (C₁-C₆)Alkyl darstellt;
R_{d} Wasserstoff oder (C₁-C₄)Alkyl darstellt;
Rₑ Wasserstoff oder (C₁-C₄)Alkyl darstellt;
m 1 oder 2 ist; und
R₁ Wasserstoff, Methyl von Ethyl darstellt.

3. Verfahren gemäß Anspruch 1-2 für die Herstellung einer Imidazolylverbindung der allgemeinen Formel 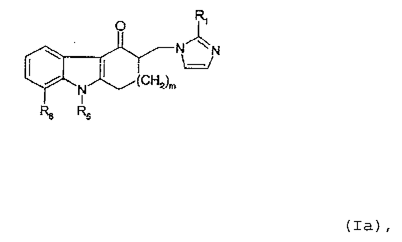 worin:
m 1 oder 2 ist;
R₁ Wasserstoff, Methyl oder Ethyl darstellt; und
R₅ ein (C₁-C₄)Alkyl darstellt
R₆ ein Wasserstoff oder ein (C₁-C₄)Alkyl darstellt oder
R₅ und R₆ zusammen mit den dazwischenliegenden Atomen einen 5-, 6- oder 7-gliedrigen Ring bilden, gegebenenfalls substituiert mit einem oder zwei Substituenten, ausgewählt aus der Gruppe bestehend aus Halogen, Hydroxyl, (C₁-C₄)Alkyl, (C₁-C₄)Alkoxyalkyl und (C₁-C₄)Alkoxy, als auch ihres pharmazeutisch annehmbaren Säureadditionssalzes;
**dadurch gekennzeichnet, dass** eine Verbindung der allgemeinen Formel 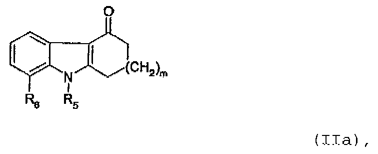 worin R₅, R₆ und m die oben definierten Bedeutungen haben; umgesetzt wird mit einer Verbindung der Formel 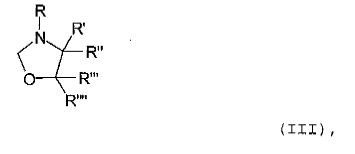 worin R, R', R", R‴ und R"" die gleichen Bedeutungen wie in Anspruch 1 haben;
gefolgt von einer Umsetzung mit einer Verbindung der Formel 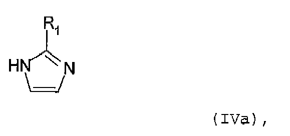 worin R₁ die gleiche Bedeutung wie in Anspruch 1 hat.

4. Verfahren gemäß Ansprüchen 1-2, **dadurch gekennzeichnet, dass** R, R', R", R‴ und R"" in Formel (III) für 2-Hydroxyethyl, Wasserstoff, Wasserstoff, Wasserstoff und Wasserstoff stehen.

5. Verfahren wie in allen vorhergehenden Ansprüchen beansprucht, **dadurch gekennzeichnet, dass** m = 1 und dass R₅ und R₆ zusammen mit den dazwischenliegenden Atomen einen 6-gliedrigen Ring bilden.

6. Verfahren wie in Ansprüchen 1-4 beansprucht, **dadurch gekennzeichnet, dass** m = 1, dass R₅ Methyl darstellt und dass R₆ Wasserstoff darstellt.

7. Verfahren wie in einem der vorhergehenden Ansprüchen beansprucht, **dadurch gekennzeichnet, dass** die Umsetzung in einem alkoholischen Lösungsmittel durchgeführt wird.

8. Verfahren wie in Anspruch 7 beansprucht, **dadurch gekennzeichnet, dass** das alkoholische Lösungsmittel 1-Butanol ist.

9. Verfahren wie in Ansprüchen 1-6 beansprucht, **dadurch gekennzeichnet, dass** die Umsetzung in einem Gemisch aus einem alkoholischen Lösungsmittel und einem aromatischen Kohlenwasserstoff durchgeführt wird.

10. Verfahren wie in Anspruch 9 beansprucht, **dadurch gekennzeichnet, dass** besagtes Gemisch ein Gemisch aus Methanol und Chlorbenzol ist.

## Revendications

1. Procédé de préparation d'un composé d'imidazolyle répondant à la formule générale : 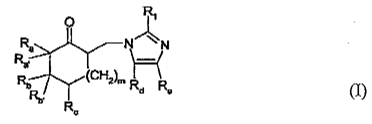 dans laquelle :
Rₐ et R_{b} représentent chacun séparément un groupe alkyle en C₁ à C₆, alcoxyalkyle en C₁ à C₆, aryle ou hétéroaryle facultativement substitué ;
ou dans laquelle Rₐ et R_{b} forment ensemble un autre système homocyclique ou hétérocyclique comprenant un ou plusieurs cycles ;
Rₐ, et R_{b}, représentent chacun l'hydrogène ou forment conjointement une double liaison carbone-carbone, ladite double liaison carbone-carbone faisant facultativement partie d'un système aromatique ;
R_{c} représente l'hydrogène, un groupe alkyle en C₁ à C₆, alcoxy en C₁ à C₆, alcoxyalkyle en C₁ à C₆ ou un halogène ;
R_{d} représente l'hydrogène ou un groupe alkyle en C₁ à C₄ ;
R_{c} représente l'hydrogène ou un groupe alkyle en C₁ à C₄ ;
m vaut 1 ou 2 ; et
R₁ représente l'hydrogène ou un groupe alkyle en C₁ à C₄ ;
ainsi que son sel d'addition d'acide ;
**caractérisé en ce qu'**un composé de formule générale : 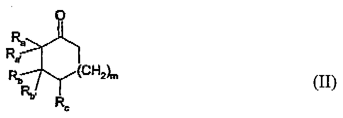 dans laquelle Rₐ, R_{a'}, R_{b} et R_{b'} ont les significations définies ci-dessus ;
est amené à réagir avec un composé de formule : 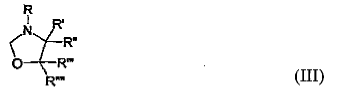 dans laquelle :
R représente l'hydrogène, un groupe alkyle en C₁ à C₄ facultativement substitué par un groupe hydroxy ou un groupe aryle facultativement substitué,
R', R", R‴ et R"" représentent chacun individuellement l'hydrogène ou un groupe alkyle en C₁ à C₄ ;
puis à réagir avec un composé de formule : 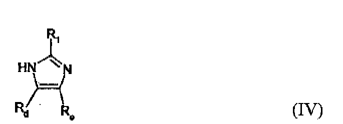
dans laquelle R₁, R_{d} et Rₑ ont les significations définies ci-dessus ;
et à réagir ensuite facultativement avec un acide approprié.

2. Procédé selon la revendication 1, dans lequel
Rₐ, R_{b}, R_{a'}, R_{b'}, R, R', R" , R"' et R"" ont les mêmes significations que dans la revendication 1 ;
Rₑ représente l'hydrogène ou un groupe alkyle en C₁ à C₆ ;
R_{d} représente l'hydrogène ou un groupe alkyle en C₁ à C₄ ;
Rₑ représente l'hydrogène ou un groupe alkyle en C₁ à C₄ ;
M vaut 1 ou 2 ; et
R₁ représente l'hydrogène, un groupe méthyle ou éthyle.

3. Procédé selon la revendication 1 ou 2, pour la préparation d'un composé d'imidazolyle répondant à la formule générale : 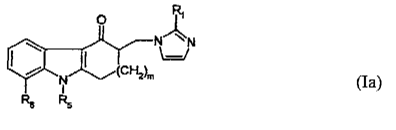 dans laquelle :
m vaut 1 ou 2 ;
R₁ représente l'hydrogène, un groupe méthyle ou éthyle ; et
R₅ est un groupe alkyle en C₁ à C₄ ;
R₆ représente l'hydrogène ou un groupe alkyle en C₁ à C₄, ou bien
R₅ et R₆, conjointement avec les atomes intermédiaires, forment un cycle à 5, 6 ou 7 chaînons, facultativement substitués par un ou deux substituants choisis dans le groupe constitué par un halogène, un groupe hydroxyle, alkyle en C₁ à C₄, alcoxyalkyle en C₁ à C₄ et alcoxy en C₁ à C₄ ;
ainsi que son sel d'addition d'acide pharmaceutiquement acceptable ;
**caractérisé en ce qu'**un composé répondant à la formule générale : 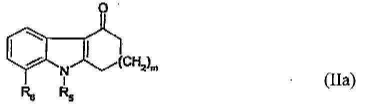 dans laquelle R₅, R₆ et m ont les significations définies ci-dessus ;
est amené à réagir avec un composé de formule : 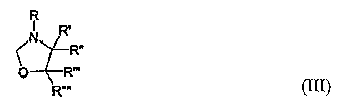 dans laquelle R, R', R", R'" et R"" ont les mêmes significations que dans la revendication 1 ;
puis à réagir avec un composé de formule : 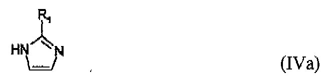 dans laquelle R₁ a la même signification que dans la revendication 1.

4. Procédé selon les revendications 1-2, **caractérisé en ce que** les radicaux R, R', R", R"' et R"" dans la formule (III) sont des radicaux 2-hydroxyéthyle, hydrogène, hydrogène, hydrogène et hydrogène, respectivement.

5. Procédé selon l'une quelconque des revendications précédentes, **caractérisé en ce que** m=1 et que les radicaux R₅ et R₆, conjointement avec les atomes intermédiaires, forment un cycle à 6 chaînons.

6. Procédé selon les revendications 1-4, **caractérisé en ce que** m=1, que R₅ est un groupe méthyle et que R₆ représente l'hydrogène.

7. Procédé selon l'une quelconque des revendications précédentes, **caractérisé en ce que** la réaction est mise en oeuvre dans un solvant alcoolique.

8. Procédé selon la revendication 7, **caractérisé en ce que** le solvant alcoolique est le 1-butanol.

9. Procédé selon les revendications 1-6, **caractérisé en ce que** la réaction est mise en oeuvre dans un mélange d'un solvant alcoolique et d'un hydrocarbure aromatique.

10. Procédé selon la revendication 9, **caractérisé en ce que** ledit mélange est un mélange de méthanol et de chlorobenzène.
